# EUROPÄISCHE PATENTSCHRIFT

(11) **EP 3 484 560 B1**
(45) Veröffentlichungstag und Bekanntmachung des Hinweises auf die Patenterteilung: **05.05.2021**
(21) Anmeldenummer: 17732925.7
(22) Anmeldetag: 27.06.2017
(51) Int. Cl.: A61M 11/00, A61M 11/02, A61M 15/00, A61M 16/00, A61M 16/14, A61M 16/20

(54) **ATEMGESTEUERTE APPLIKATION VON PULVERFÖRMIGEM AEROSOL BEI DER BEATMUNG ODER ATEMUNTERSTÜTZUNG EINES PATIENTEN**
RESPIRATION-CONTROLLED APPLICATION OF AEROSOL IN POWDER FORM DURING THE ARTIFICIAL RESPIRATION OR SUPPORTED RESPIRATION OF A PATIENT
SYSTÈME D'APPLICATION, À COMMANDE PAR RESPIRATION, D'AÉROSOL EN POUDRE PENDANT LA RESPIRATION ARTIFICIELLE OU L'ASSISTANCE RESPIRATOIRE D'UN PATIENT

(30) Priorität: 13.07.2016 DE 102016112822
(43) Veröffentlichungstag der Anmeldung: 22.05.2019
(73) Patentinhaber: Fraunhofer-Gesellschaft zur Förderung der angewandten Forschung e.V., 80686 München (DE); MTF MediTech Franken GmbH, 90542 Eckental (DE)
(72) Erfinder: POHLMANN, Gerhard, 31715 Meerbeck (DE); WIEGANDT,Felix Carl, 30529 Hannover (DE); IWATSCHENKO, Peter, 90542 Eckenthal (DE)
(74) Vertreter: Raffay & Fleck
(86) Internationale Anmeldenummer: PCT/EP2017/065878
(87) Internationale Veröffentlichungsnummer: WO 2018/010954

(56) Entgegenhaltungen:
- WO-A1-2006/108558
- WO-A1-2009/042187
- WO-A1-2010/122103
- WO-A1-2011/004275
- WO-A2-2005/048982
- DE-A1-102008 050 218
- US-A1- 2005 229 926

## Beschreibung

Die vorliegende Erfindung betrifft ein System zur atemgesteuerten Applikation von pulverförmigem Aerosol bei der Beatmung oder Atemunterstützung eines Patienten.

Unter Beatmung wird die teil- oder vollständige Übernahme der physiologischen Atmung durch externe Hilfen oder Quellen verstanden. Sie wird bei unzureichender Spontanatmung oder deren kompletten Ausfall durchgeführt. Hingegen meint Atemunterstützung die Unterstützung der Spontanatmung, bei der ein kontinuierlicher Überdruck über den gesamten Atemzyklus angelegt wird (CPAP, Continuous Positive Airway Pressure). Der Patient kann hierbei seine Atemtiefe, Atemfrequenz und auch den Luftdurchfluss selbst bestimmen. Voraussetzung für die Anwendung der Atemunterstützung ist also die prinzipielle Fähigkeit des Patienten zur eigenen Atmung.

So besteht z.B. bei der lokalen Behandlung von Lungeninfektionen (bakteriell, viral oder mit Mykosen) mit Wirkstoffen ein erheblicher medizinischer Bedarf an derartigen Systemen und Verfahren, insbesondere auch bei beatmeten oder atemunterstützten Patienten. Im Besonderen besteht dieser Bedarf bei der Behandlung des sogenannten Atemnotsyndroms bei Früh- und Neugeborenen (IRDS, Infant Respiratory Distress Syndrome), bei dem die unreife Lunge nur in geringem Maße oder gar nicht sog. Surfactant (eine spezielle oberflächenaktive Substanz in der Lunge) produziert. Dadurch kollabieren Lungenbläschen, die dann am Gasaustausch nicht teilnehmen können. Sauerstoffmangel, Atemnot und ggf. Lungenschäden sind die Folgen. Zur Behandlung wird das Frühgeborene oder Neugeborene künstlich beatmet oder atemunterstützt, wobei Surfactant in die Lunge eingebracht werden muss. Dies erfolgt zurzeit invasiv per Instillation einer Surfactantsuspension.

Hierbei liegt der lokale pulmonale Wirkstoffbedarf für die inhalative Applikation mit bis zu einigen 100 mg Wirkstoff pro Tag 2 bis 3 Größenordnungen über dem der "klassischen" inhalativen Applikation. Dabei besteht ein dringender Bedarf in der Weiterentwicklung sowohl von portablen und stationären, vom Patienten zu verwendenden Inhalationsgeräten als insbesondere auch in der Entwicklung von Aerosoldosierverfahren zur Kopplung mit intensivmedizinischen Beatmungstechnologien.

Insbesondere die Verkürzung von Anwendungszeiten durch höhere Dosierraten an der Patientenschnittstelle stellt bei chronischen Erkrankungen einen hohen medizinischen Bedarf dar. Die Dispergierung von Pulvern ist hier ein vielversprechender Weg. Hierbei ist aber eine optimale Verzahnung von Aerosolisier- und Beatmungstechnik wichtig.

Insbesondere bei Kindern erfordern die fehlende Koordinationsfähigkeit sowie die sich im Laufe der Kindesentwicklung ändernde Lungenanatomie und Atemmechanik die Entwicklung automatisierter sich an die individuellen Gegebenheiten anpassender Systeme. Daher ist eine enge Kopplung zwischen der sensorischen in-situ-Atemüberwachung und der Steuerung der zu applizierenden Dosis notwendig. Generell ist die atemgetriggerte Vernebelung von Flüssigkeiten bereits weit verbreitet (Clinical Foundations Nr. 14, S.1.-6, Arzu Ari:"Aerosol Therapy for Ventilator-Dependent Patients: Devices, Issues, Selection & Technique"; Clinical Foundations Nr. 14 S. 7-12, Panel Discussion, Moderator Timm Myers: "Aerosol Therapy in spontaneously Breathing and mechanically ventilated patient: Description, Selection & Issues").

Aber auch Pulverinhalatoren werden zunehmend beschrieben. So offenbart beispielsweise die WO 2010/122 103 A1 ein System zur Applikation von pulverförmigem Aerosol in einem Beatmungssystem, wobei das pulverförmige Aerosol kontinuierlich zudosiert wird. Bei dieser Art der Zudosierung wird allerdings ein größerer Teil des pulverförmigen Aerosols verbraucht, ohne dass dieser in den tiefen Lungenarealen deponiert wird und dort seine volle Wirkung entfalten kann. Somit ist eine derartige Betriebsweise teuer und uneffizient.

In der DE 10 2008 050 218 A1 ist ein System und ist ein Verfahren zur Applikation inhalierbarer Stoffe in eine Lunge offenbart. Das System ist dazu vorgesehen mit einer Tierlunge oder einer post-mortem entnommenen humanen Lunge verbunden zu werden. So kann das Verfahren an solchen nicht in einem lebendigen Organismus integrierten Lunge zum Testen neuer Wirkstoffe verwendet werden.

Die US 2005/0229926 A1 offenbart ein Verfahren und eine Zusammensetzung zur Behandlung von Lungenerkrankungen. Das dort offenbarte Verfahren und ein zu dessen Durchführung gezeigtes System arbeiten mit einem Flüssigaerosol, nicht jedoch mit Pulveraerosol.

Die WO 2005/048982 A2 offenbart ein System zum Verabreichen eines Pulveraerosols in den Atemgasstrom eines Patienten. Dort wird in einem Aerosolerzeuger generiertes Aerosol direkt in den Atemgasstrom eingeleitet.

Der Erfindung liegt die Aufgabe zugrunde, ein System zur atemgesteuerten Applikation von pulverförmigem Aerosol derart zu modifizieren, dass die Therapieeffizienz erhöht wird, bei einem gleichzeitig einfachen und kostengünstigen Aufbau.

Diese Aufgabe wird gelöst durch ein System zur atemgesteuerten Applikation von pulverförmigem Aerosol bei der Beatmung oder Atemunterstützung eines Patienten mit den Merkmalen des Anspruchs 1. Vorteilhafte Weiterbildungen eines solchen Systems sind in den abhängigen Ansprüchen 2 bis 12 bezeichnet. Einen besonderen Aspekt der Erfindung bildet ein in dem erfindungsgemäßen System zur atemgesteuerten Applikation von pulverförmigem Aerosol bei der Beatmung oder Atemunterstützung eines Patienten eingesetztesVentil mit den in den abhängigen Anspruch 10 näher bezeichneten Merkmalen. Vorteilhafte Weiterbildungen eines ein solches Ventil enthaltenden Systems sind in den abhängigen Ansprüchen 11-12 bezeichnet. Beschrieben wird auch ein mit dem vorliegenden Patent jedoch nicht geschütztes Verfahren zur atemgesteuerten Applikation von pulverförmigem Aerosol bei der Beatmung oder Atemunterstützung eines Patienten.

Somit zielt die Erfindung auf die atemgesteuerte Applikation von pulverförmigem Aerosol in einem Kreislauf zur Zufuhr von Atemgas, insbesondere bei einer Atemunterstützung durch CPAP, aber auch bei einer Beatmung. Die Anwendung der Erfindung betrifft besonders Früh- und Neugeborene sowie Kleinkinder, aber ebenso Heranwachsende und Erwachsene, wie z. B. solche mit einer chronisch obstruktiven Lungenerkrankung (COPD).

Bei einem entsprechenden System beinhaltend eine Schnittstelle, die mit den Atemwegen des zu beatmenden oder atemzuunterstützenden Patienten in Kontakt bringbar ist, ein Gerät zum Erzeugen eines Atemgasstroms, wobei der Atemgasstrom einen ersten Druck aufweist, der höher oder gleich dem Umgebungsdruck ist, mindestens eine Inspirationsleitung, durch die der Atemgasstrom zu der Schnittstelle geleitet wird, einen Aerosolerzeuger, mindestens eine Aerosolleitung, durch die das erzeugte pulverförmige Aerosol zu der Schnittstelle geleitet wird und einen Atemsensor, der das Atemsignal des Patienten erfasst, besteht nun der erfindungsgemäße Ansatz darin, das pulverförmige Aerosol vor der Applikation direkt an der Schnittstelle vorzuhalten und nach Bedarf zuzuführen. Die Zufuhr wird über ein Ventil geregelt, das in der mindestens einen Aerosolleitung zwischen Schnittstelle und Aerosolerzeuger angeordnet ist. Dazu wird das Ventil in Abhängigkeit des erfassten Atemsignals gesteuert und das erzeugte pulverförmige Aerosol in einem Zwischenspeicher bevorratet, der zwischen dem Ventil und dem Aerosolerzeuger angeordnet ist, wobei das in dem Zwischenspeicher bevorratete pulverförmige Aerosol einen durch eine Steuerung vorgegebenen zweiten Druck aufweist, der größer oder gleich dem ersten Druck ist, insbesondere 2-3 mbar größer sein kann. So wird das pulverförmige Aerosol direkt dem Einatemgasstrom zugemischt oder ersetzt diesen für kurze Zeit sogar völlig, wenn das Ventil geöffnet wird.

Pulverförmiges Aerosol bezeichnet ein heterogenes Gemisch (Dispersion) aus festen Schwebeteilchen in einem Gas. Die festen Schwebeteilchen resultieren aus einem inhalierbaren Trockenpulver, das sowohl trocken appliziert werden kann als auch vor der Applikation befeuchtet werden kann, um insbesondere bei inhalierbaren Trockenpulvern aufweisend hygroskopische Eigenschaften gewährleisten zu können, dass diese die Lunge nicht austrocknen. Dabei kann das pulverförmige Aerosol insbesondere einen maximalen Partikeldurchmesser von 4 *µ*m aufweisen.

Das Atemsignal kann sich aus den Phasen Einatmen, Ausatmen und Pause zusammensetzen. Bei der Beatmung werden die maschinellen Beatmungshübe der Zielgröße entsprechend entweder volumenkontrolliert (Volume Controlled Ventilation, VCV) oder druckkontrolliert (Pressure ControlledVentilation, PCV) verabreicht. Der Atemsensor kann das Atemsignal über die Bewegung des Brustkorbs/der Bauchdecke, über die Temperatur an der Schnittstelle, über die Strömung an der Schnittstelle und/oder über den Druck an der Schnittstelle bestimmen. Bei der Beatmung kann das Atemsignal auch über das Gerät zum Erzeugen eines Atemgasstroms erfasst werden. In diesem Fall kann das Gerät zum Erzeugen eines Atemgasstroms den Atemsensor enthalten.

Für die Steuerung des Ventils wird das erfasste Atemsignal des Atemsensors ausgewertet und der Verlauf des Atemzyklus bestimmt. Zu einem vorgegebenen Zeitpunkt innerhalb eines Atemzyklus, während dessen das pulverförmige Aerosol verabreicht werden soll, wird dann das Ventil geöffnet.

Hierbei kann ein Gerät zum Erzeugen eines Atemgasstroms einen Ventilator, eine Druckgasflasche oder dergleichen umfassen.

Ein Aerosolerzeuger kann z. B. eine Vorrichtung gemäß der WO 2010/122 103 A1 umfassen.

Das erzeugte pulverförmige Aerosol kann sowohl direkt über die mindestens eine Aerosolleitung zu der Schnittstelle geleitet werden als auch indirekt über die mindestens eine Inspirationsleitung, wobei das erzeugte pulverförmige Aerosol durch die mindestens eine Aerosolleitung zu der mindestens einen Inspirationsleitung geleitet wird und in dieser weiter zu der Schnittstelle geleitet wird.

Eine Schnittstelle kann eine Atemmaske, einen Tubus, ein Mundstück, eine Nasenkanüle, einen Prong oder dgl. umfassen. Hierbei kann die Schnittstelle einen Anschluss für die mindestens eine Inspirationsleitung aufweisen. Des Weiteren kann die Schnittstelle einen Anschluss für die mindestens eine Aerosolleitung aufweisen und/oder einen Anschluss für eine Exspirationsleitung.

Die Exspirationsleitung kann z. B. den Exspirationsluftstrom des Patienten zurück zu dem Gerät zum Erzeugen eines Atemgasstroms leiten. Dabei kann die Exspirationsleitung einen oder mehrere Filter aufweisen, um die im Exspirationsluftstrom enthaltenen Pulverpartikel des pulverförmigen Aerosols abzuscheiden, sodass dieses nicht in das Gerät zur Erzeugung eines Atemgasstroms eindringt und dessen Funktion beeinträchtigt.

Auch kann die Schnittstelle eine weitere Leitung für eine gesonderte Abführung der pulverförmiges Aerosol enthaltenden Atemluft aufweisen, wobei diese weitere Leitung ein Auslassventil aufweisen kann. Insbesondere kann auch das Auslassventil in Abhängigkeit des vom Atemsensor erfassten Atemsignals gesteuert werden, sodass das Ventil während der Inspiration des Patienten geöffnet ist und das Auslassventil geschlossen ist bzw. das Ventil während der Exspiration des Patienten geschlossen ist und das Auslassventil geöffnet ist. Auf diese Weise kann das Totvolumen innerhalb der Schnittstelle, also das Volumen der Exspirationsluft, die nach der Exspiration des Patienten noch in der Schnittstelle verbleibt, weiter minimiert werden. Hierbei ist es von Vorteil, wenn das Auslassventil direkt an der Schnittstelle angeschlossen ist. Im Besonderen kann das Auslassventil den gleichen Aufbau und die gleiche Funktionsweise aufweisen wie das Ventil. Auch kann die mindestens eine Inspirationsleitung zusätzlich einen oder mehrere Filter und/oder Ventile, Rückschlagklappen oder dergleichen zwischen dem Gerät zum Erzeugen des Atemgasstroms, der Schnittstelle und/oder dem Ort der Zugabe des erzeugten pulverförmigen Aerosols aufweisen.

Auch ist es von Vorteil, wenn sich das Ventil direkt an der Schnittstelle oder unmittelbar in deren Bereich befindet und so klein ist, dass es die Funktion der Schnittstelle nicht beeinträchtigt, d. h. die Bedienbarkeit und die Positionierung der Schnittstelle am Patienten darf nicht durch die Größe und das Gewicht des Ventils beeinflusst werden. Gleichzeitig sollte das Ventil aber im geöffneten Zustand das erzeugte pulverförmige Aerosol optimal durchlassen. Hierfür kann das Ventil im geöffneten Zustand eine Apertur aufweisen, die dem Querschnitt der mindestens einen Aerosolleitung entspricht. Auch ist es von Vorteil, wenn das Ventil keine abrupten Übergänge, wie z. B. Kanten aufweist.

Weiter kann das Ventil eine Schaltzeit von kleiner als 20 ms aufweisen, um einen optimalen Dosierzeitpunkt des pulverförmigen Aerosols zu realisieren.

Um Totzeiten bzw. Totvolumina zwischen Aerosolerzeuger und Schnittstelle weiter zu reduzieren, kann das Leitungsvolumen zwischen Ventil und Schnittstelle kleiner als das Tidalvolumen des Patienten sein, insbesondere kann das Leitungsvolumen zwischen Ventil und Schnittstelle maximal 1/10 des Tidalvolumens des Patienten betragen.

Hierbei meint Totvolumen das Volumen, das zunächst mit dem pulverförmigen Aerosol nach dem Öffnen des Ventils zu befüllen ist, bis dieses zu der Schnittstelle gelangt. Totzeit bezeichnet die Zeitdauer, nach der das pulverförmige Aerosol nach dem Öffnen des Ventils die Schnittstelle erreicht.

Für das Tidalvolumen bzw. die Atemfrequenz sind folgende Richtwerte maßgebend (Quellen: Walsh,B.K. et al "Perinatal and pediatric respiratory care evolve learning system" 3. Auflage, ISBN 978-1-4160-2448-4 bzw. Philip Chi Lip Kwok, Hak-Kim Chan "Delivery of inhalation drugs to children for asthma and other respiratory diseases" Advanced Drug Delivery Reviews 73 (2014) S. 83- 88):

| | Altersklassifikation | Tidalvolumen [ml/kg] | Atemfrequenz [Atemzüge/Mi- |
|---|---|---|---|
| Erwachsener | ≥ 12 Jahre | 7-10 | 12-20 |
| Heranwachsender | 6 - 12 Jahre | 7-10 | 18-25 |
| Kind | 2 - 6 Jahre | 6-9 | 20-30 |
| Kleinkind | 1 Monat - 2 Jahre | 5-8 | 20-35 |
| Neugeborenes | 0 - 30 Tage | 5-8 | 25-40 |
| Frühgeborenes | Gestationsalter < 38 | 4-6 | 25-60 |

Um stets eine ausreichende Menge an pulverförmigem Aerosol bereitzustellen, kann die Summe des Volumens des Zwischenspeichers und des Leitungsvolumens zwischen Zwischenspeicher und Ventil größer sein als das Tidalvolumen des Patienten, insbesondere kann die Summe des Volumens des Zwischenspeichers und des Leitungsvolumens zwischen Zwischenspeicher und Ventil wenigstens das Zweifache des Tidalvolumens des Patienten betragen.

Des Weiteren ist es von Vorteil, wenn der Zwischenspeicher mindestens eine elastische Wand aufweist, um so über einen konstanten Druck des im Zwischenspeicher bevorrateten pulverförmigen Aerosols einen gleichbleibenden Zugabestrom des pulverförmigen Aerosols über die gesamte Öffnungsdauer des Ventils zu gewährleisten.

Um stets eine ausreichende Bereitstellung des pulverförmigen Aerosols im Zwischenspeicher zu realisieren, kann der Aerosolerzeuger in Abhängigkeit des im Zwischenspeicher vorliegenden zweiten Druckes gesteuert werden.

In einer vorteilhaften Weiterbildung kann das Ventil umfassen, einen Kanal mit Anschlusselementen und mehreren Verschlusselementen, wobei die Verschlusselemente flexible Wandsegmente aufweisen und auf der Innenwand des Kanals entlang eines Umfangabschnittes radial gleichmäßig verteilt angeordnet sind, sodass diese jeweils mit der Innenwand des Kanals Steuerräume ausbilden, die untereinander zu einem Gesamtvolumensystem verbunden sind und die mit einem Steuerfluid beaufschlagbar sind, sodass die Verschlusselemente aufgrund einer Druckerhöhung des in den Steuerräumen befindlichen Steuerfluids in den Querschnitt des Kanals und gegeneinander gequetscht werden.

Insbesondere kann das Ventil drei, vier oder fünf radial symmetrisch verteilte, identisch ausgebildete Verschlusselemente umfassen, wobei das Volumen der Steuerräume der Verschlusselemente gleich ist. So verformen sich die flexiblen Wandsegmente durch die Erhöhung des Druckes des Steuerfluids backenförmig und sperren somit die Aerosolleitung ab. Durch die Aufteilung des Steuerraums in mehrere insbesondere drei, vier oder fünf auf das Volumen bezogen gleichgroße Verschlusselemente kann eine besonders hohe Symmetrie innerhalb des Ventils während des geschlossenen Zustandes erreicht werden.

Weiter ist es von Vorteil, wenn die Verschlusssegmente als Elastomermembran-Schlauchmanschette ausgeführt sind, die im Kanal angeordnet ist, und deren beiden Enden druckdicht festgelegt sind, wobei die Elastomermembran-Schlauchmanschette über den Umfang gleichmäßig verteilt entlang der Länge mehrere Verstärkungen aufweist und zwischen den Verstärkungen die flexiblen Wandsegmente ausgebildet sind, sodass zwischen der Innenwand des Kanals und der Außenwand der Elastomermembran-Schlauchmanschette mehrere Steuerräume gebildet werden. Insbesondere können drei, vier oder fünf radial symmetrisch verteilte, identisch ausgebildete Steuerräume zwischen der Innenwand des Kanals und der Außenwand der Elastomermembran-Schlauchmanschette gebildet werden, wobei das Volumen der Steuerräume gleich groß ist. Im Besonderen kann die Elastomermembran-Schlauchmanschette als Silikonmembran-Schlauchmanschette ausgeführt sein.

In einer besonderen vorteilhaften Variante kann das Gesamtvolumensystem ein Gesamtvolumen aufweisen, das maximal 1/3 des Tidalvolumens des Patienten beträgt, um bei einer Ruptur der flexiblen Wandsegmente sicherstellen zu können, dass nur ein sehr kleines Volumen des Steuerfluids in die Aerosolleitung und somit in die Atemwege des zu beatmenden oder atemzuunterstützenden Patienten gelangt. Insbesondere kann das Gesamtvolumen maximal 0,7 ml betragen, wenn es sich bei dem Patienten um ein Frühgeborenes handelt. Hierbei kann das Ventil einen Innendurchmesser von 5 mm und eine Länge von 10 mm aufweisen. Handelt es sich bei dem Patienten um einen Erwachsenen, kann das Gesamtvolumen maximal 14 ml betragen. Hierbei kann das Ventil einen Innendurchmesser von 19 mm und eine Länge von 50 mm haben. Im Besonderen kann das Gesamtvolumensystem durch einen beweglichen Kolben oder ein flexibles Element abgeschlossen sein, wobei durch die Bewegung des Kolbens oder die Kompression des flexiblen Elements der Druck in dem mit Steuerfluid beaufschlagten Gesamtvolumensystem erhöht wird.

Ein Ventil mit den oben dargelegten (und optionalen) Eigenschaften kann auch als Ventil zum Einsatz in einem System zur atemgesteuerten Applikation von pulverförmigem Aerosol bei der Beatmung oder Atemunterstützung eines Patienten zum Einsatz kommen. Da das Ventil in unmittelbarem Kontakt zum Patienten steht, kann das Ventil insbesondere als Einwegartikel konzipiert sein, der nach der Verwendung entsorgt wird.

In einem von dem Patentschutz nicht erfassten weiteren Aspekt wird ein Verfahren zur atemgesteuerten Applikation von pulverförmigem Aerosol bei der Beatmung oder Atemunterstützung eines Patienten bereitgestellt. Dieses beinhaltet die folgenden Schritte: Erzeugen eines Atemgasstroms, der einen ersten Druck aufweist, der höher oder gleich dem Umgebungsdruck ist; Leiten des Atemgasstroms zu einer Schnittstelle, die mit den Atemwegen eines zu beatmenden oder atemzuunterstützenden Patienten in Kontakt steht; Erzeugen von pulverförmigem Aerosol; Leiten des pulverförmigen Aerosols zu der Schnittstelle; Erfassen eines Atemsignals, wobei das erzeugte pulverförmige Aerosol zwischen dem Ort der Erzeugung von pulverförmigem Aerosol und der Schnittstelle in einem Zwischenspeicher bevorratet wird, wobei das im Zwischenspeicher bevorratete pulverförmige Aerosol einen zweiten Druck aufweist, der größer oder gleich dem ersten Druck ist, insbesondere 2-3 mbar größer sein kann, und wobei die Zugabe des erzeugten pulverförmigen Aerosols in Abhängigkeit des erfassten Atemsignals über ein Ventil geregelt wird, das sich zwischen Zwischenspeicher und Schnittstelle befindet.

Die Dosierung des pulverförmigen Aerosols kann in dem erfindungsgemäßen System über die Zugabedauer und die Konzentration des im Zwischenspeicher bevorraten pulverförmigen Aerosol eingestellt werden. Des Weiteren kann über den Zugabezeitpunkt der Wirkort des pulverförmigen Aerosols eingestellt werden, d. h., in welche Lungenareale das pulverförmige Aerosol gelangt. So gelanget das pulverförmige Aerosol bspw. bei einer Zugabe direkt zu Beginn des Atemzyklus, also zu Beginn des Einatmens, bis in die tiefen Lungenareale. Insbesondere kann das pulverförmige Aerosol als Bolus innerhalb des Atemzyklus hinzugegeben werden.

Ein Einsatz des erfindungsgemäßen Systems ist auch bei einer Beatmung bzw. Atemunterstützung mit einem einen erhöhten Sauerstoffgehalt aufweisenden Atemgas möglich.

So ermöglicht das erfindungsgemäße System eine optimale Zudosierung des pulverförmigen Aerosols und somit eine hohe Therapieeffizienz, sodass die Anwendungszeiten oder benötigte Arzneimittelmengen erheblich verringert werden können. Bspw. kann abgeschätzt werden, dass sich die applizierte Menge bei einem IRDS-Patienten um ca. 60% reduzieren lässt.

Weitere Vorteile und Merkmale der Erfindung ergeben sich aus der nachstehenden Beschreibung mehrerer Ausführungsbeispiele anhand der beigefügten Figuren. Dabei zeigt:
- Figur 1: eine schematische Darstellung des erfindungsgemäßen Systems zur atemgesteuerten Applikation von pulverförmi-gem Aerosol bei der Atemunterstützung eines Patienten (CPAP-Verfahren)
- Figur 2: eine schematische Längeschnitt Darstellung eines erfindungsgemäßen Ventils im geöffneten Zustand
- Figur 3: eine schematische Längeschnitt Darstellung eines erfindungsgemäßen Ventils im geschlossenen Zustand
- Figur 4: eine schematische Querschnitt Darstellung eines erfindungsgemäßen Ventils im geöffneten Zustand
- Figur 5: eine schematische Querschnitt Darstellung eines erfindungsgemäßen Ventils im geschlossenen Zustand
- Figur 6: eine schematische Darstellung einer möglichen Elastomermembran-Schlauchmanschette im geöffneten Zustand des erfindungsgemäßen Ventils
- Figur 7: eine schematische Darstellung einer möglichen Elastomermembran-Schlauchmanschette im geschlossenen Zustand des erfindungsgemäßen Ventils

- Figur 8: beispielhaft die zeitliche Platzierung des pulverförmigen Aerosol unter Anwendung eines Systems gemäß der vorliegenden Erfindung

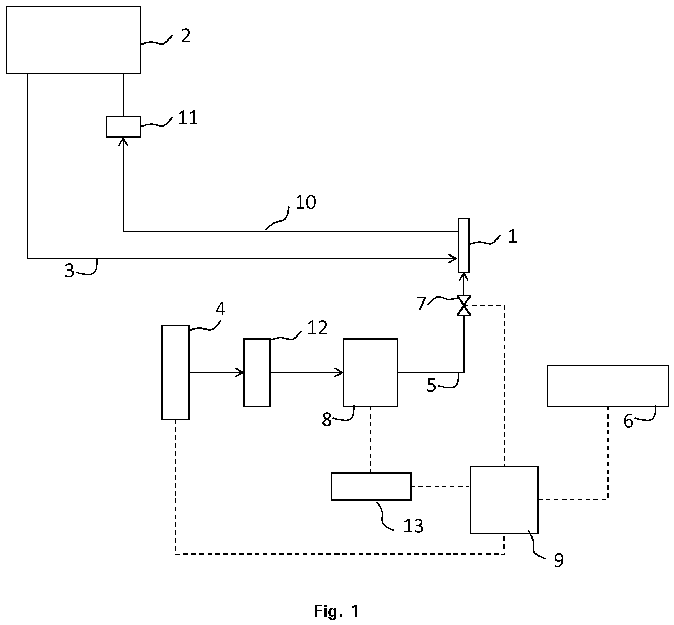

In Figur 1 sind verschiedene Komponenten des erfindungsgemäßen Systems dargestellt: eine Schnittstelle 1, die mit den Atemwegen des atemzuunterstützenden Patienten in Kontakt steht, ein Gerät zum Erzeugen eines Atemgasstroms 2, eine Inspirationsleitung 3, durch die der Atemgasstrom zu der Schnittstelle 1 geleitet wird, ein Aerosolerzeuger 4, eine Aerosolleitung 5, durch die das erzeugte pulverförmige Aerosol zu der Schnittstelle 1 geleitet wird, ein Atemsensor 6, ein Ventil 7, sowie ein Zwischenspeicher 8 zum Bevorraten des erzeugten pulverförmigen Aerosols, ein Befeuchter 12 zum Befeuchten des pulverförmigen Aerosols, eine Steuerung 9 zur Steuerung des Ventils und des Aerosolerzeugers, ein Drucksensor 13 zur Erfassung des im Zwischenspeicher vorliegenden Drucks, sowie eine Exspirationsleitung 10 aufweisend einen Filter 11.

Das Gerät zur Erzeugung eines Atemgasstroms 2 stellt einen Atemgasstrom über die Inspirationsleitung 3 an der Schnittstelle 1 bereit. Das im Aerosolerzeuger 4 erzeugte pulverförmige Aerosol wird ggf. im Befeuchter 12 befeuchtet und über die Aerosolleitung 5 zu der Schnittstelle 1 geleitet. Das Ventil 7 befindet sich in der Aerosolleitung 5 zwischen Zwischenspeicher 8 und Schnittstelle 1 und wird über die Steuerung 9 gesteuert, die das erfasste Atemsignal des Atemsensors 6 auswertet. Die Steuerung des Aerosolerzeugers 3 z. B. erfolgt in Abhängigkeit des vorliegenden Druckes 13 des pulverförmigen Aerosols im Zwischenspeicher 8. Der Exspirationsluftstrom des Patienten wird über die Exspirationsleitung 10 zu dem Gerät zur Erzeugung eines Atemgasstroms 2 geleitet, wobei die im Exspirationsluftstrom enthaltene Pulverpartikel des pulverförmigen Aerosols zunächst in dem Filter 11 abgetrennt werden, sodass diese das Gerät zum Erzeugen eines Atemgasstroms 2 nicht in seiner Funktion beeinträchtigen.

Das pulverförmige Aerosol wird im Aerosolerzeuger 4 erzeugt, bis der im Zwischenspeicher 8 vorliegende Druck dem durch die Steuerung 9 vorgegeben Druck entspricht. Das Ventil 7 ist geschlossen. Der Patient wird kontinuierlich durch einen Atemgasstrom atemunterstützt, der durch das Gerät zur Erzeugung eines Atemgasstroms 2 bereitgestellt wird. Der Atemsensor 6 erfasst das Atemsignal des Patienten und leitet dieses an die Steuerung 9 weiter, die es auswertet. Zu einem vorgegebenen Zeitpunkt innerhalb des Atemzyklus, während dessen das pulverförmige Aerosol zudosiert werden soll, wird das Ventil 7 aufgrund eines entsprechenden Signals der Steuerung 9 geöffnet und so das pulverförmige Aerosol direkt dem Atemgasstrom zugemischt oder das pulverförmige Aerosol ersetzt diesen sogar für kurze Zeit. Nach Ablauf der voreingestellten Zugabedauer wird das Ventil 7 durch ein Signal der Steuerung 9 wieder geschlossen. Hierbei beträgt die Schaltzeit des Ventils weniger als 20 ms, um einen optimalen Dosierzeitpunkt realisieren zu können.

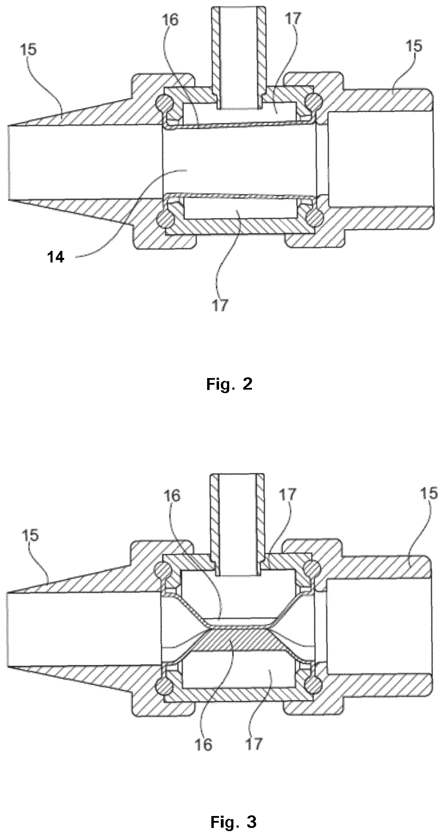

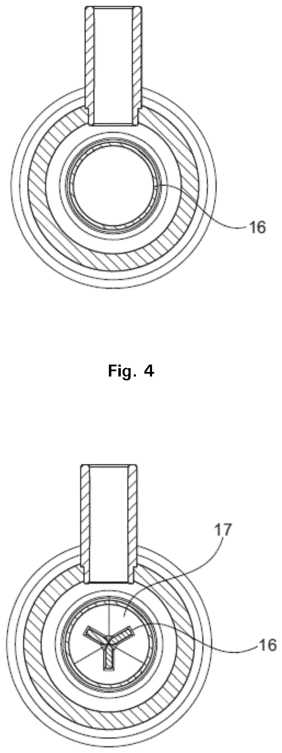

Figur 2 zeigt die verschiedenen Komponenten eines erfindungsgemäßen Ventils im geöffneten Zustand des Ventils: einen Kanal 12, Anschlusselemente 13 flexible Wandsegmente 14, Steuerräume 15, die untereinander zu einem geschlossenen Gesamtvolumensystem verbunden sind, das mit Steuerfluid beaufschlagt ist, wobei die flexiblen Wandsegmente reversibel in Abhängigkeit von dem in den Steuerräumen anliegenden Druck des Steuerfluids verformbar sind. Durch eine Erhöhung des Druckes des im Steuerraum 15 vorliegenden Steuerfluids durch einen beweglichen Kolben, der das System abschließt, werden die flexiblen Wandsegmente 14 backenförmig verformt und derart in den Querschnitt des Kanals und gegeneinander gequetscht, dass das erzeugte pulverförmige Aerosol dicht abgesperrt wird (Figur 3 und 5). Wird der im Steuerraum 15 vorliegende Druck des Steuerfluids verringert, öffnen sich die flexiblen Wandsegemente14 wieder zum vollen Durchgang (Figur 4). In einem Anwendungsfall für ein Früh- oder Neugeborenes kann das Gesamtvolumen des Gesamtvolumensystems 0,7 ml betragen. Hierbei kann das Ventil einen Innendurchmesser von 5 mm und eine Länge von 10 mm haben. In einem Anwendungsfall für einen Erwachsenen kann das Gesamtvolumen 14 ml betragen. Hierbei kann das Ventil einen Innendurchmesser von 19 mm und eine Länge von 50 mm haben.

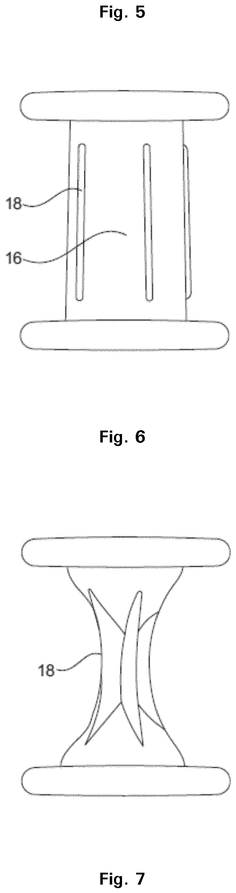

Figur 6 und 7 zeigen ein besonders vorteilhaftes Ausführungsbeispiel der Verschlusselemente als Elastomermembran-Schlauchmanschette, die im Kanal angeordnet ist, und deren beiden Enden druckdicht festgelegt sind, wobei die Elastomermembran-Schlauchmanschette über den Umfang gleichmäßig verteilt entlang der Länge mehrere Verstärkungen 18 aufweist und zwischen den Verstärkungen die flexiblen Wandsegmente 16 ausgebildet sind. So verformt sich, bei einer Implementation der Elastomermembran-Schlauchmanschette in das Ventil, die Elastormermembran-Schlauchmanschette in dem Bereich der Verstärkungen 18 durch eine Erhöhung des Druckes des im Steuerraum vorliegenden Steuerfluids wesentlich geringer im Vergleich zu den flexiblen Wandsegmenten 16, dem zwischen den Vestärkungen18 liegendem Bereich. Die flexiblen Wandsegmente 16 werden backenförmig verformt und so in den Querschnitt des Kanals gequetscht, dass die Leitung dicht abgesperrt wird.

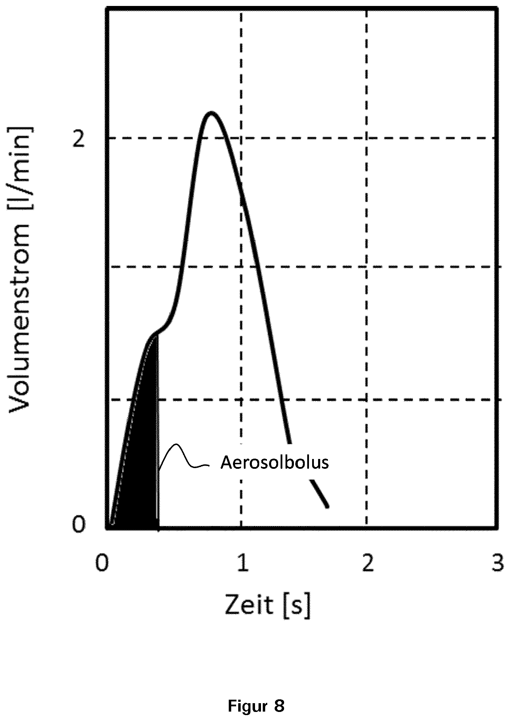

Figur 8 zeigt beispielhaft die zeitliche Platzierung der Wirkstoffzugabe mittels eines erfindungsgemäßen Systems während einer Inhalationsphase. Das pulverförmige Aerosol wird hier z. B. direkt zu Beginn der Inhalationsphase zugegeben, sodass auch die tiefen Lungenareale erreicht werden können. Hierbei kann über das Ventil die Dauer und der Zeitpunkt der Zugabe eines Aerosolbolus eingestellt werden. Das zugegebene Volumen an pulverförmigem Aerosol kann 20-30% des Tidalvolumens des Patienten betragen.

### Bezugszeichenliste

- 1: Schnittstelle
- 2: Gerät zum Erzeugen eines Atemgasstroms
- 3: Inspirationsleitung
- 4: Aerosolerzeuger
- 5: Aerosolleitung
- 6: Atemsensor
- 7: Ventil
- 8: Zwischenspeicher
- 9: Steuerung
- 10: Exspirationsleitung
- 11: Filter
- 12: Befeuchter
- 13: Drucksensor
- 14: Kanal
- 15: Anschlusselemente
- 16: flexible Wandsegmente
- 17: Steuerraum
- 18: Verstärkungen

## Patentansprüche

1. System zur atemgesteuerten Applikation von pulverförmigem Aerosol bei der Beatmung oder Atemunterstützung eines Patienten beinhaltend eine Schnittstelle (1), die mit den Atemwegen des zu beatmenden oder atemzuunterstützenden Patienten in Kontakt bringbar ist,
ein Gerät zum Erzeugen eines Atemgasstroms (2), wobei der Atemgasstrom einen ersten Druck aufweist, der höher oder gleich dem Umgebungsdruck ist,
mindestens eine Inspirationsleitung (3), durch die der Atemgasstrom zu der Schnittstelle (1) geleitet wird,
einen Aerosolerzeuger (4),
mindestens eine Aerosolleitung (5), durch die das erzeugte pulverförmige Aerosol vom Aerosolerzeuger (4) zu der Schnittstelle (1) geleitet wird, und einen Atemsensor (6), der das Atemsignal des zu beatmenden oder atemzuunterstützenden Patienten erfasst, wobei ein Ventil (7) in der mindestens einen Aerosolleitung (5) zwischen Schnittstelle (1) und Aerosolerzeuger (4) angeordnet ist, das in Abhängigkeit des erfassten Atemsignals gesteuert wird,
**dadurch gekennzeichnet, dass**
ein Zwischenspeicher (8) zum Bevorraten von erzeugtem pulverförmigem Aerosol zwischen dem Ventil (7) und dem Aerosolerzeuger (4) angeordnet ist, wobei eine Steuerung (9) vorgesehen und dazu eingerichtet ist, einen zweiten Druck vorzugeben, der größer oder gleich dem ersten Druck ist, und zu veranlassen, dass im Aerosolerzeuger 4 Aerosol erzeugt und in den Zwischenspeicher (8) gegeben wird, bis der im Zwischenspeicher (8) vorliegende Druck dem durch die Steuerung (9) vorgegeben zweiten Druck entspricht,
wobei aufgrund der Steuerung des Ventils (7) das im Zwischenspeicher (8) bevorratete pulverförmige Aerosol dem Atemgasstrom direkt zumischbar ist.

2. System nach Anspruch 1 **dadurch gekennzeichnet, dass** das Ventil (7) im geöffneten Zustand eine Apertur aufweist, die dem Querschnitt der mindestens einen Aerosolleitung (5) entspricht.

3. System nach einem der vorangehenden Ansprüche, **dadurch gekennzeichnet, dass** das Ventil (7) eine Schaltzeit kleiner als 20 ms aufweist.

4. System nach einem der vorangehenden Ansprüche, **dadurch gekennzeichnet, dass** das Leitungsvolumen zwischen Ventil (7) und Schnittstelle (1) kleiner als das Tidalvolumen des Patienten ist.

5. System nach Anspruch 4, **dadurch gekennzeichnet, dass** das Leitungsvolumen zwischen Ventil (7) und Schnittstelle (1) maximal 1/10 des Tidalvolumens des Patienten beträgt.

6. System nach einem der vorangehenden Ansprüche, **dadurch gekennzeichnet, dass** die Summe des Volumens des Zwischenspeichers (8) und des Leitungsvolumens zwischen Zwischenspeicher (8) und Ventil (7) größer ist als das Tidalvolumen des Patienten.

7. System nach Anspruch 6, **dadurch gekennzeichnet, dass** die Summe des Volumens des Zwischenspeichers (8) und des Leitungsvolumens zwischen Zwischenspeicher (8) und Ventil (7) wenigstens das Zweifache des Tidalvolumens des Patienten beträgt.

8. System nach einem der vorangehenden Ansprüche, **dadurch gekennzeichnet, dass** der Zwischenspeicher (8) mindestens eine elastische Wand aufweist.

9. System nach einem der vorangehenden Ansprüche, **dadurch gekennzeichnet, dass** der Aerosolerzeuger (4) in Abhängigkeit des im Zwischenspeicher (8) vorliegenden zweiten Druckes gesteuert wird.

10. System nach einem der vorangehenden Ansprüche **dadurch gekennzeichnet, dass** das Ventil (7) einen Kanal (14) mit Anschlusselementen (15) und mehreren Verschlusselementen beinhaltet, wobei die Verschlusselemente flexible Wandsegmente (16) aufweisen und auf der Innenwand des Kanals (14) entlang eines Umfangabschnittes radial gleichmäßig verteilt angeordnet sind, sodass diese jeweils mit der Innenwand des Kanals (14) Steuerräume (17) ausbilden, die untereinander zu einem Gesamtvolumensystem verbunden sind und die mit Steuerfluid beaufschlagbar sind, sodass die Verschlusselemente aufgrund einer Druckerhöhung des in den Steuerräumen (17) befindlichen Steuerfluids in den Querschnitt des Kanals und gegeneinander gequetscht werden.

11. System nach Anspruch 10, **dadurch gekennzeichnet, dass** das Ventil (7) drei, vier oder fünf radial symmetrisch verteilte, identisch ausgebildete Verschlusselemente umfasst, wobei das Volumen der Steuerräume (17) der Verschlusselemente gleich groß ist.

12. System nach Anspruch 10 oder 11 **dadurch gekennzeichnet, dass** das Gesamtvolumensystem ein Gesamtvolumen aufweist, das maximal 1/3 des Tidalvolumens des Patienten beträgt.

## Claims

1. System for respiration-controlled application of aerosol in powder form during ventilation or respiratory support of a patient, comprising an interface (1) which can be brought into contact with the airways of the patient to be ventilated or to receive respiratory support,
a device for generating a respiratory gas stream (2), the respiratory gas stream having a first pressure that is higher than or equal to the ambient pressure,
at least one inspiration line (3) through which the respiratory gas flow is directed to the interface (1),
an aerosol generator (4),
at least one aerosol line (5) through which the aerosol in powder form produced is conducted from the aerosol generator (4) to the interface (1), and a respiratory sensor (6) which detects the respiratory signal of the patient to be ventilated or to receive respiratory support, a valve (7) being arranged in said at least one aerosol line (5) between the interface (1) and the aerosol generator (4), which valve is controlled as a function of the respiratory signal detected,
**characterised in that**
an intermediate reservoir (8) for storing generated aerosol in powder form is arranged between the valve (7) and the aerosol generator (4), wherein a controller (9) is provided and arranged to preset a second pressure which is greater than or equal to the first pressure and to cause aerosol to be generated in the aerosol generator (4) and to be supplied to the intermediate reservoir (8) until the pressure present in the intermediate reservoir (8) corresponds to the second pressure preset by the controller (9),
wherein, due to the control of the valve (7), the aerosol in powder form stored in the intermediate storage (8) can be mixed directly with the respiratory gas flow.

2. A system according to claim 1, **characterised in that** the valve (7) in the open state has an aperture which corresponds to the cross-section of said at least one aerosol line (5).

3. A system according to one of the preceding claims, **characterised in that** the valve (7) has a switching time of less than 20 ms.

4. A system according to any of the preceding claims, **characterised in that** the line volume between valve (7) and interface (1) is smaller than the patient's tidal volume.

5. A system according to claim 4, **characterised in that** the line volume between valve (7) and interface (1) is at most 1/10 of the patient's tidal volume.

6. A system according to any one of the preceding claims, **characterised in that** the sum of the volume of the intermediate reservoir (8) and the conduit volume between the intermediate reservoir (8) and the valve (7) is greater than the patient's tidal volume.

7. A system according to claim 6, **characterised in that** the sum of the volume of the intermediate reservoir (8) and the line volume between the intermediate reservoir (8) and the valve (7) is at least twice the tidal volume of the patient.

8. A system according to one of the preceding claims, **characterised in that** the intermediate reservoir (8) has at least one elastic wall.

9. A system according to one of the preceding claims, **characterised in that** the aerosol generator (4) is controlled as a function of the second pressure present in the intermediate reservoir (8).

10. A system according to one of the preceding claims, **characterised in that** the valve (7) includes a duct (14) with connection elements (15) and a plurality of closure elements, the closure elements having flexible wall segments (16) and being arranged on the inner wall of the duct (14) in a radially uniformly distributed manner along a circumferential section, so that they each form control spaces (17) with the inner wall of the duct (14), which are connected to one another to form a total volume system and which can be acted upon by control fluid, so that the closure elements are squeezed into the cross-section of the duct and against one another as a result of an increase in pressure of the control fluid located in the control spaces (17).

11. A system according to claim 10, **characterised in that** the valve (7) comprises three, four or five identically formed closure elements distributed radially symmetrically, the volume of the control spaces (17) of the closure elements being equal.

12. A system according to claim 10 or 11 **characterised in that** the total volume system has a total volume which is at most 1/3 of the patient's tidal volume.

## Revendications

1. Système d'application à commande par respiration d'un aérosol sous forme de poudre pendant la ventilation ou l'assistance respiratoire d'un patient, comprenant
une interface (1) qui peut être mise en contact avec les voies respiratoires du patient à ventiler ou à recevoir une assistance respiratoire,
un dispositif pour générer un flux de gaz respiratoire (2), le flux de gaz respiratoire ayant une première pression qui est supérieure ou égale à la pression ambiante,
au moins une ligne d'inspiration (3) par laquelle le flux de gaz respiratoire est dirigé vers l'interface (1),
un générateur d'aérosol (4),
au moins une ligne d'aérosol (5) par laquelle l'aérosol sous forme de poudre produit est conduit du générateur d'aérosol (4) à l'interface (1), et un capteur respiratoire (6) qui détecte le signal respiratoire du patient à ventiler ou à recevoir une assistance respiratoire, une valve (7) étant disposée dans ladite au moins une ligne d'aérosol (5) entre l'interface (1) et le générateur d'aérosol (4), laquelle valve est commandée en fonction du signal respiratoire détecté,
**caractérisé en ce que**
un réservoir intermédiaire (8) pour stocker l'aérosol généré sous forme de poudre est disposé entre la valve (7) et le générateur d'aérosol (4), dans lequel un dispositif de commande (9) est prévu et disposé pour prérégler une seconde pression qui est supérieure ou égale à la première pression et pour faire en sorte que l'aérosol soit généré dans le générateur d'aérosol (4) et soit fourni au réservoir intermédiaire (8) jusqu'à ce que la pression présente dans le réservoir intermédiaire (8) corresponde à la seconde pression préréglée par le dispositif de commande (9),
dans lequel, grâce à la commande de la valve (7), l'aérosol sous forme de poudre stocké dans le stockage intermédiaire (8) peut être mélangé directement avec le flux de gaz respiratoire.

2. Système selon la revendication 1, **caractérisé en ce que** la valve (7) à l'état ouvert a une ouverture qui correspond à la section transversale de ladite au moins une ligne d'aérosol (5).

3. Système selon l'une des revendications précédentes, **caractérisé en ce que** la vanne (7) a un temps de commutation inférieur à 20 ms.

4. Système selon l'une quelconque des revendications précédentes, **caractérisé en ce que** le volume de la conduite entre la valve (7) et l'interface (1) est inférieur au volume courant du patient.

5. Système selon la revendication 4, **caractérisé en ce que** le volume de la ligne entre la valve (7) et l'interface (1) est au plus égal à 1/10 du volume courant du patient.

6. Système selon l'une quelconque des revendications précédentes, **caractérisé en ce que** la somme du volume du réservoir intermédiaire (8) et du volume du conduit entre le réservoir intermédiaire (8) et la valve (7) est supérieure au volume courant du patient.

7. Système selon la revendication 6, **caractérisé en ce que** la somme du volume du réservoir intermédiaire (8) et du volume de la conduite entre le réservoir intermédiaire (8) et la valve (7) est au moins deux fois le volume courant du patient.

8. Système selon l'une des revendications précédentes, **caractérisé en ce que** le réservoir intermédiaire (8) comporte au moins une paroi élastique.

9. Système selon l'une des revendications précédentes, **caractérisé en ce que** le générateur d'aérosol (4) est commandé en fonction de la seconde pression présente dans le réservoir intermédiaire (8).

10. Système selon l'une des revendications précédentes, **caractérisé en ce que** la soupape (7) comprend un canal (14) avec des éléments de raccordement (15) et une pluralité d'éléments de fermeture, les éléments de fermeture présentant des segments de paroi flexibles (16) et étant disposés sur la paroi intérieure du canal (14) de manière répartie radialement de façon uniforme le long d'une section circonférentielle, de sorte qu'ils forment respectivement avec la paroi intérieure du canal (14) des espaces de commande (17) qui sont reliés entre eux pour former un système de volume total et qui peuvent être sollicités par un fluide de commande, de sorte que les éléments de fermeture sont comprimés dans la section transversale du canal et les uns contre les autres par une augmentation de la pression du fluide de commande se trouvant dans les espaces de commande (17).

11. Système selon la revendication 10, **caractérisé en ce que** la vanne (7) comprend trois, quatre ou cinq éléments de fermeture de forme identique répartis de manière radialement symétrique, le volume des espaces de commande (17) des éléments de fermeture étant égal.

12. Système selon les revendications 10 ou 11, **caractérisé en ce que** le système de volume total a un volume total qui est au plus égal à 1/3 du volume courant du patient.
